(19) Europäisches Patentamt
European Patent Office
Office européen des brevets

(11) Veröffentlichungsnummer: **0 254 856 B1**

# (12) EUROPÄISCHE PATENTSCHRIFT

(45) Veröffentlichungstag der Patentschrift: **04.09.91**

(51) Int. Cl.5: **C07D 213/38**, C07D 213/61, C07D 409/12, A61K 31/44

(21) Anmeldenummer: **87108706.0**

(22) Anmeldetag: **16.06.87**

Die Akte enthält technische Angaben, die nach dem Eingang der Anmeldung eingereicht wurden und die nicht in dieser Patentschrift enthalten sind.

## (54) Pyridinäthanolaminderivate.

(30) Priorität: **27.06.86 CH 2608/86**
**27.03.87 CH 1186/87**

(43) Veröffentlichungstag der Anmeldung:
**03.02.88 Patentblatt 88/05**

(45) Bekanntmachung des Hinweises auf die Patenterteilung:
**04.09.91 Patentblatt 91/36**

(84) Benannte Vertragsstaaten:
**AT BE CH DE ES FR GB GR IT LI LU NL SE**

(56) Entgegenhaltungen:
**EP-A- 0 006 735**
**EP-A- 0 023 385**
**EP-A- 0 070 133**
**EP-A- 0 099 707**
**EP-A- 0 101 069**
**EP-A- 0 139 921**
**EP-A- 0 164 700**
**EP-A- 0 236 624**
**US-A- 4 358 455**

(73) Patentinhaber: **F. HOFFMANN-LA ROCHE AG**
**Postfach 3255**
**CH-4002 Basel(CH)**

(72) Erfinder: **Alig, Leo, Dr.**
**Liebrütistrasse 32**
**CH-4303 Kaiseraugst(CH)**
Erfinder: **Müller, Marcel, Dr.**
**Quellenweg 10**
**CH-4402 Frenkendorf(CH)**

(74) Vertreter: **Lederer, Franz, Dr. et al**
**Patentanwalt Dr. Franz Lederer Lucile-Grahn-Strasse 22**
**W-8000 München 80(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue Pyridinäthanolaminderivate, Verfahren zu ihrer Herstellung und pharmazeutische Präparate auf der Basis dieser Verbindungen.

Die erfindungsgemässen Pyridinäthanolaminderivate sind Verbindungen der Formel

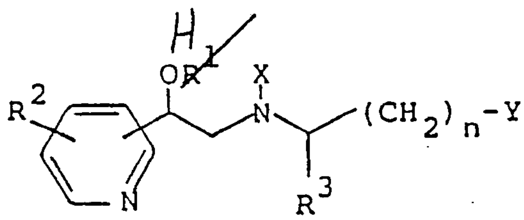

I

worin

| | |
|---|---|
| n | 1 oder 2 |
| X | H, nieder-Alkyl, nieder-Alkoxy-nieder-alkyl oder eine Gruppe $X^a$ der Formel |

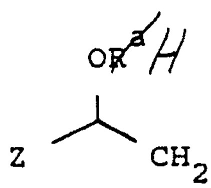

$X^a$

| | |
|---|---|
| Z | eine Gruppe der Formel |

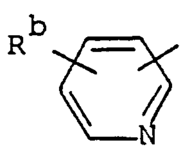

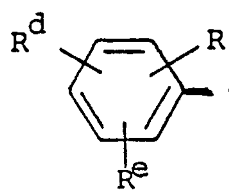

oder

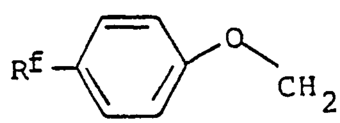

Z

($Z^1$) ($Z^2$) ($Z^3$)

Y eine Gruppe

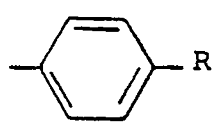

| | |
|---|---|
| R | eine Gruppe $COR^4$, $C(R^5)=CHCOR^4$ oder OR" |
| R" | H, $C_{1-4}$-Alkyl, $(CH_2)_{1-6}$-OH, $(CH_2)_{1-6}$-$O(CH_2)_{1-6}$-$R^6$ oder $(CH_2)_{1-6}$-$COR^4$ |
| $R^2$ und $R^b$ | H, Cl oder Br |
| $R^3$ und $R^5$ | H oder $CH_3$ |
| $R^4$ | Hydroxy, $C_{1-4}$-Alkoxy oder $NH_2$ |
| $R^6$ | H, $R^9$, OH oder $COR^4$ |
| $R^c$ und $R^e$ | H oder Cl, |
| $R^d$ | H oder $NH_2$ |
| $R^f$ | H, $CH_3CONH$, $NH_2COCH_2$ oder $R^9CH_2CH_2OCH_2CH_2O$ |
| $R^9$ und $R^9$ | Phenyl sind, |

sowie die physiologisch verträglichen Salze davon.

Beispiele von $C_{1-4}$-Alkyl- und -Alkoxygruppen sind Methyl, Aethyl, Propyl, Isopropyl, n-Butyl und

Isobutyl; Methoxy, Aethoxy, Propoxy, Isopropoxy, Butoxy und Isobutoxy.

Die Verbindungen der Formel I bilden mit Säuren Salze, die ebenfalls Gegenstand der Erfindung sind. Beispiele solcher Salze sind Salze mit physiologisch verträglichen Mineralsäuren, wie Salzsäure, Bromwasserstoffsäure, Schwefelsäure, Phosphorsäure; oder mit organischen Säuren, wie Oxalsäure, Methansulfonsäure, Essigsäure, Propionsäure, Zitronensäure, Maleinsäure, Bernsteinsäure, Aepfelsäure, Fumarsäure, Phenylessigsäure oder Salicylsäure.

Die erfindungsgemässen Verbindungen enthalten zumindest ein asymmetrisches Kohlenstoffatom und können somit als optisch aktive Enantiomere, als Diastereomere oder als Racemate vorliegen.

Die Verbindungen der Formel I, worin ein in der Gruppe Y vorliegender Rest $R^4$ $C_{1-4}$-Alkoxy oder $NH_2$ ist, sowie diejenigen Verbindungen, worin n die Zahl 1 und $R^2$ Chlor in 6-Stellung eines 2-Pyridylrestes ist, sind bevorzugt. Bevorzugt sind ferner diejenigen Verbindungen, worin X Wasserstoff oder eine Gruppe $X^a$; Z 6-Chlor-2-pyridyl, und Y in p-Stellung durch 2-Aethoxyäthoxy, 2-Phenäthoxyäthoxy oder Methoxycarbonylmethoxy substituiertes Phenyl ist. Ebenfalls bevorzugt sind diejenigen Verbindungen, worin X eine Gruppe $X^a$; Z in p-Stellung durch Carbamoylmethyl, Acetamid oder 2-Phenäthoxyäthoxy substituiertes Phenoxymethyl, und Y p-(2-Aethoxyäthoxy)phenyl ist.

Unter den obigen Verbindungen sind diejenigen, worin $R^3$ Wasserstoff oder Methyl mit R-Konfiguration ist, besonders bevorzugt. Beispiele von solchen Verbindungen sind:

p-[(R)-2-[[ (R)-2-(6-Chlor-2-pyridyl)-2-hydroxyäthyl]amino]propyl]benzoesäuremethylester,

p-[(R)-2-[[(R)-2-(6-Chlor-2-pyridyl)-2-hydroxyäthyl][(S)-m-chlor-β-hydroxyphenäthyl]amino]propyl]-benzoesäuremethylester,

α,α'-[[[(R)-p-(2-Aethoxyäthoxy)-α-methylphenäthyl]imino]dimethylen]bis[(RS)-6-chlor-2-pyridinmethanol],

(RS)-6-Chlor-α-[[[(R)-p-(2-äthoxyäthoxy)-α-methylphenäthyl]amino]methyl]-2-pyridinmethanol,

α,α'-[[[p-(2-Aethoxyäthoxy)phenäthyl]imino]dimethylen]bis[(RS)-6-chlor-2-pyridinmethanol,

(R)-6-Brom-α-[[[(RS)-2-(6-brom-2-pyridyl)-2-hydroxyäthyl][(R)-p-(2-äthoxyäthoxy)-α-methylphenäthyl]-amino]methyl]-2-pyrimidinmethanol,

(R)-6-Chlor-α-[[[(S)-2-(6-chlor-2-pyridyl)-2-hydroxyäthyl][(R)-α-methyl-p-(2-phenäthoxyäthoxy)-phenäthyl]amino]methyl]-2-pyridinmethanol,

2-[p-[(RS)-3-[[(RS)-2-(6-Chlor-2-pyridyl)-2-hydroxyäthyl][p-(2-äthoxyäthoxy)phenäthyl]amino]-2-hydroxypropoxy]phenyl]acetamid,

4'-[(RS)-3-[[(RS)-2-(6-Chlor-2-pyridyl)-2-hydroxyäthyl][(R)-P-(2-äthoxyäthoxy)-α-methylphenäthyl]amino]-2-hydroxypropoxy]acetanilid und

6-Chlor-α-[[[(R)-p-(2-äthoxyäthoxy)-α-methylphenäthyl][(RS)-2-hydroxy-3-[p-[2-(phenäthoxy)äthoxy]-phenoxy]propyl]amino]metnyl]-2-pyridinmethanol.

Die erfindungsgemässen Verbindungen können dadurch hergestellt werden, dass man

a) ein Amin der Formel

$(X^1,X^2)NC(H,R^3)(CH_2)_nY$ (II)

worin eines von $X^1$ und $X^2$ Wasserstoff ist und das andere eine der Bedeutungen von X hat oder die Gruppe der Formel

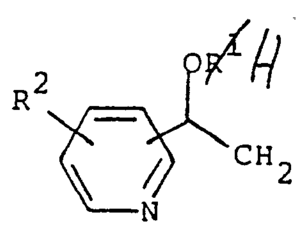

$X^3$

ist, mit einem die Gruppe $X^3$ oder eine der Gruppen X einführenden Mittel alkyliert und

b) gewünschtenfalls einen in einer Gruppe Y des Reaktionsproduktes enthaltenen reaktionsfähigen Substituenten funktionell abwandelt und gewünschtenfalls eine Verbindung der Formel I in ein Salz überführt.

Beispiele von beim Verfahren a) verwendbaren Alkylierungsmittel sind Verbindungen der Formel QT, Z° $CHOHCH_2T$, Z° $COCH_2T$ oder

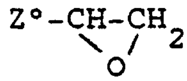

III

worin

Q eine der Gruppen X oder $X^3$,

Z° eine Gruppe Z oder

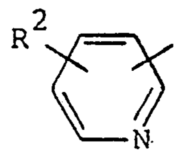

($Z^4$)

und

T Halogen, insbesondere Brom oder Chlor, oder eine Sulfonatgruppe, wie Methansulfonat,

ist.

Die Alkylierung a) kann man in an sich bekannter Weise bewerkstelligen, z.B. wie beschrieben in den europäischen Patentanmeldungen 101069A1 und 140243A1, zweckmässig unter Erhitzen in einem geeigneten Lösungsmittel. So lässt man ein Amin II und ein Epoxyd III, vorzugsweise unter einer inerten Atmosphäre, wie Argon, in einem inerten organischen Lösungsmittel, z.B. Dimethylsulfoxid (DMSO), Acetonitril oder einem Aether, wie Tetrahydrofuran (THF) oder Dioxan, oder einem Alkohol, wie Aethanol, bei einer Temperatur zwischen 60° C und dem Siedepunkt des Reaktionsgemisches reagieren. Wenn man an Stelle des Epoxyds ein Halogenid Z°$CHOHCH_2T$ oder Z°$COCH_2T$ einsetzt, kann man in einem inerten organischen Lösungsmittel, wie einem halogenierten Kohlenwasserstoff, z.B. Chloroform bei einer Temperatur bis zu 200° C arbeiten. Bei Einsatz eines nieder-Alkylhalogenids QT kann man in einem Lösungsmittel, wie Acetonitril, in Gegenwart einer Base, wie Natriumcarbonat, bei einer Temperatur bis zu 60° C arbeiten. Bei Einsatz eines Halogenids der Formel Z°$COCH_2T$ entsteht ein Zwischenprodukt, in dem die Ketogruppe Z°CO zur Alkoholgruppe Z°CHOH reduziert werden muss. Diese Reaktion kann man mit einem komplexen Metallhydrid, wie $NaBH_4$, in einem Lösungsmittel, wie einem Alkanol, z.B. Methanol, bei etwa 20-30° C durchführen.

Gewünschtenfalls kann man einen in einer Gruppe Y des Reaktionsproduktes der Formel I enthaltenen reaktionsfähigen Substituenten in an sich bekannter Weise funktionell abwandeln. So kann beispielsweise ein Phenol der Formel I, worin R Hydroxy ist, mit einem eine Gruppe R″ einführenden Mittel umgesetzt werden. Beispiele von solchen Mitteln sind Verbindungen der Formel TR″, worin T und R″ die obige Bedeutung haben. Diese Umsetzung kann man in an sich bekannter Weise durchführen, z.B. in einem Lösungsmittel, wie DMSO, Aceton, THF oder n-Propanol, in Gegenwart einer Base, wie Kaliumhydroxyd, Kaliumcarbonat, Kalium-t-butylat oder Triäthylamin, gegebenenfalls unter Argon bei einer Temperatur bis zur Rückflusstemperatur des Reaktionsgemisches.

Ein in der Gruppe Y vorliegender nieder-Carbalkoxyrest kann in an sich bekannter Weise zum Carboxyrest hydrolysiert werden, z.B. mit einer Säure, wie Salzsäure, Schwefelsäure oder Phosphorsäure, oder mit einer Base, wie einem Alkalimetallhydroxyd, zweckmässig bei einer Temperatur bis zu etwa 110° C und in einem Lösungsmittel, wie Wasser oder einem nieder-Alkanol, z.B. Methanol oder Aethanol, bei der sauren Hydrolyse, bzw. einem wässrigen nieder-Alkanol bei der basischen Hydrolyse.

Die Amine der Formel II und die beim Verfahren a) verwendbaren Alkylierungsmittel können, soweit sie nicht bekannte Verbindungen sind, in an sich bekannter Weise hergestellt werden. So kann man ein Epoxyäthylpyridin III durch Reaktion des entsprechenden Pyridincarbaldehyds mit Trimethylsulfoniummethylid in flüssigem Ammoniak herstellen.

Die erfindungsgemässen Pyridinäthanolaminderivate können als Wirkstoffe in pharmazeutischen Präparaten zur Behandlung der Fettsucht und/oder des Diabetes mellitus, insbesondere des obesen erwachsenen Diabetikers verwendet werden. Im Tierexperiment wurde auf Verabreichung der erfindungsgemässen Pyridinäthanolaminderivate ein gesteigerter Katabolismus, vor allem der Fette, beobachtet. Weiterhin wurde beobachtet, dass die erfindungsgemässen Pyridinäthanolaminderivate die Bildung von braunem Fettgewebe bei Ratten und obes-hyperglykämischen Mäusen stimulieren. Bekanntlich wird Defekten des braunen

Fettgewebes eine wesentliche Rolle bei der Entstehung der Fettsucht zugeschrieben. An obes-hyperglykämischen Mäusen und an Streptozotocin-diabetischen Ratten haben die erfindungsgemässen Pyridinäthanolaminderivate einen ausgeprägten antidiabetischen Effekt, indem sie hypoglykämisch wirken und die Glykosurie vermindern. Die erfindungsgemässen Pyridinäthanolaminderivate zeigen nur eine geringe Wirkung auf Herztätigkeit und Kreislauf. Die Dosierung kann in Abhängigkeit von der Wirkungsstärke der einzelnen Verbindungen und den individuellen Bedürfnissen des Patienten 0,5-1000 mg, vorzugsweise 2-200 mg pro Tag für einen Erwachsenen betragen, wobei die Dosis als Einzeldosis oder in mehreren Dosen über den Tag verteilt verabreicht werden kann.

Ferner konnte mit den erfindungsgemässen Pyridinäthanolaminderivaten im Tierexperiment eine Erhöhung des Proteingehaltes und eine Erniedrigung des Fettgehalts im Körper nachgewiesen werden. Die erfindungsgemässen Pyridinäthanolaminderivate führen demnach zu einer Erhöhung der mageren Körpermasse auf Kosten des Fettanteils. Daher können die erfindungsgemässen Pyridinäthanolaminderivate in der Humanmedizin zur Behandlung von Zuständen, die mit erhöhtem Proteinabbau verbunden sind, z.B. bei Rekonvaleszenz nach Operationen, verwendet werden. Dabei sind die Verabreichungsdosen die gleichen wie bei der Behandlung der Fettsucht und/oder des Diabetes mellitus.

Die erfindungsgemässen Pyridinäthanolaminderivate können auch in der Ernährung von Masttieren, wie Rindern, Schweinen, Schafen und Geflügel, Verwendung finden. Dabei können die Verabreichungsdosen und Verabreichungsformen die gleichen sein wie für Vitamine. Die erfindungsgemässen Pyridinäthanolaminderivate können auch als Futterzusatz in Dosen von 0,01-100 mg/kg je nach Substanz, Tierart und Alter eingesetzt werden.

Die pharmazeutischen Präparate enthalten den Wirkstoff zusammen mit einem verträglichen pharmazeutischen, organischen oder anorganischen Trägermaterial, wie z.B. Wasser, Gelatine, Gummi arabicum, Lactose, Stärke, Magnesiumstearat, Talk, pflanzliche Oele, Polyalkylenglykole, Vaseline und dergleichen. Die pharmazeutischen Präparate werden vorzugsweise oral, z.B. in Form von Tabletten, Kapseln, Pillen, Pulver, Granulaten, Lösungen, Sirupen, Suspensionen, Elixiren und dergleichen verabreicht. Die Verabreichung kann aber auch parenteral, z.B. in Form von sterilen Lösungen, Suspensionen oder Emulsionen, erfolgen. Die pharmazeutischen Präparate können sterilisiert sein und/oder Bestandteile enthalten, wie Konservierungsmittel, Stabilisatoren, Netzmittel, Emulgatoren, Salze, um den osmotischen Druck zu variieren, und Puffersubstanzen.

Die Aktivität der neuen Verbindungen der Formel I wird aus den nachstehenden Versuchsresultaten deutlich:

Wirkung auf den Sauerstoffverbrauch

Männliche Albinoratten im Gewicht von 160-180 g wurden nach 24 Stunden Fasten in Stoffwechselkäfige gesetzt. Die Käfige wurden mit konstant 6 Liter Raumluft/Minute, die bei einem Taupunkt von 11°C äquilibriert wurde, belüftet. Von der Abluft wurde nach erneuter Aequilibrierung während Perioden von jeweils 14 Minuten Proben gesammelt und der Sauerstoff- und $CO_2$-Gehalt analysiert. Nach einer Anpassungszeit von 4 Stunden erhielten die in Gruppen zu 6 aufgeteilten Tiere entweder Placebo (5% Gummi arabicum) oder die Testsubstanz (suspendiert in 5% Gummi arabicum) per os. Danach wurden 12 Stunden lang die Bestimmungen durchgeführt. In Tabelle I ist der Prozentsatz des gemittelten Sauerstoffverbrauchs nach Medikation während der ersten 3 Stunden und der gesamten Versuchsdauer (12 Stunden) vom Sauerstoffverbrauch der Anpassungsperiode angegeben, wobei entsprechende Korrekturen für Aenderungen in der Placebo-Gruppe berücksichtigt wurden.

Tabelle I

| Verbindung hergestellt in Beispiel No. | Dosis µM/kg | $O_2$-Verbrauch % vom Wert der Vorperiode 1.-3. Stunde | 1.-12. Stunde |
|---|---|---|---|
| 2Fb | 0,3 | 152 | 121 |
| 4B | 0,1 | 165 | 124 |
| 4C | 0,3 | 180 | 133 |
| 4Fa | 0,1 | 139 | 109 |
| 4G | 0,3 | 123 | 105 |
| 4Ia | 1 | 183 | 137 |
| 4Jb | 1 | 185 | 140 |
| 4Jc | 0,3 | 143 | 117 |
| 4Ka | 0,1 | 158 | 118 |
| 4Kb | 0,3 | 156 | 116 |
| 5a | 0,1 | 139 | 113 |
| 5b | 1 | 172 | 127 |
| 7 | 1 | 156 | 115 |
| 8A | 1 | 176 | 132 |
| 8B | 0,1 | 135 | 107 |
| 9 | 1 | 185 | 143 |
| 11 | 1 | 174 | 134 |

Beispiel 1

Eine Lösung von 1,51 g p-[(R)-2-Aminopropyl]phenol und 1,33 g 2-(Epoxyäthyl)pyridin (erhalten durch Reaktion von Pyridin-2-carbaldehyd mit Trimethylsulfoniummethylid in flüssigem Ammoniak, IR-Banden bei 3056, 3012, 1593, 1474, 1438, 1148, 995, 878, 781 $cn^{-1}$) in 40 ml DMSO wurde 24 Stunden unter Argon auf 100° erwärmt. Das Reaktionsgemisch wurde dann im Hochvakuum bei 70° zur Trockene verdampft und der Rückstand wurde auf Silicagel mit Chloroform/n-Propanol/gesättigte wässerige $NH_3$-Lösung (1000:100:5) chromatographiert. Man erhielt

a) 0,8 g α,α'-[[[(R)-p-Hydroxy-α-methylphenäthyl]imino]dimethylen]bis[(RS)-2-pyridinmethanol],

$$[\alpha]_D^{20°} = -21° \text{ (c = 0,3 in MeOH) und}$$

b) 1,4 g (RS)-α-[[[(R)-p-Hydroxy-α -methylphenäthyl]amino]methyl]-2-pyridinmethanol,

$$[\alpha]_D^{20^\circ} = -29^\circ \text{ (c = 0,5 in Methanol).}$$

Beispiel 2

Analog Beispiel 1 erhielt man

2A) aus 1,70 g 2-Chlor-6-epoxyäthylpyridin (hergestellt aus 6-Chlor-2-pyridincarbaldehyd durch Methylenierung mit Dimethylsulfoniummethylenid in flüssigem Ammoniak, IR-Banden bei 1587, 1563, 1448, 1416, 1158, 1135, 888, 798 $cm^{-1}$) und 1,51 g p-[(R)-2-Amino-propyl]phenol

a) 0,94 g α,α'-[[[(R)-p-Hydroxy-α-methylphenäthyl]imino]dimethylen]bis[(RS)-6-chlor -2-pyridinmethanol],

$$[\alpha]_D^{20^\circ} = -21^\circ \text{ (c = 0,5 in MeOH) und}$$

b) 2,3 g (RS)-α-[[[(R)-p-Hydroxy-α -methylphenäthyl]amino]methyl]-6-chlor-2-pyridinmethanol,

$$[\alpha]_D^{20^\circ} = -24^\circ \text{ (c = 0,5 in Methanol)}$$

2B) aus 1,60 g 2-Epoxyäthylpyridin und 1,50 g Tyramin

a) 0,84 g α,α'-[[[p-Hydroxyphenäthyl]imino]dimethylen]bis[(RS) -2-pyridinmethanol], IR-Banden bei 1612, 1596, 1571, 1515, 1242, 1106, 1075, 826 und 770 $cm^{-1}$ und

b) 1,34 g (RS)-α-[[[p-Hydroyphenäthyl]amino]methyl] -2-pyridinmethanol, IR-Banden bei 1612, 1594, 1571, 1515, 1250, 828, 769 $cm^{-1}$

2C) aus 1,71 g 2-Chlor-6-epoxyäthylpyridin und 1,51 g Tyramin

a) 1,1 g α,α'-[[[p-Hydroxyphenäthyl]imino]dimethylen]bis[(RS) -6-chlor-2-pyridinmethanol], IR-Banden bei 3276, 1613, 1585, 1561, 1515, 1232, 829, 798 $cm^{-1}$ und

b) 1,9 g (RS)-6-Chlor-α-[[[p-hydroxyphenäthyl]amino]methyl] -2-pyridinmethanol, IR-Banden bei 1612, 1585, 1561, 1515, 1252, 1158, 1138, 1108, 1047, 828, 798 $cm^{-1}$

2D) aus 3,0 g p-[(R)-2-Aminopropyl]phenol und 6,0 g 2-Brom-6-epoxyäthylpyridin (hergestellt durch Reaktion von 2-Brompyridin-6-carbaldehyd mit Trimethylsulfoniummethylid in flüssigem Ammoniak, IR-Banden bei 1585, 1556, 1444, 1412, 1159, 1119, 882, 796 $cm^{-1}$)

a) 7,39 g α,α'-[[[(R)-p-Hydroxy-α -methylphenäthyl]imino]dimethylen]bis[(RS)-6-brom -2-pyridinmethanol],

$$[\alpha]_D^{20^\circ} = -13^\circ \text{ (c = 0,7 in MeOH) und}$$

b) 2,0 g (RS)-α-[[[(R)-p-Hydroxy-α-methylphenäthyl]amino]methyl] -6-brom-2-pyridinmethanol,

$$[\alpha]_D^{20^\circ} = -19^\circ \text{ (c = 1,0 in MeOH)}$$

2E) aus 3,6 g 2-Chlor-6-epoxyäthylpyridin und 3,3 g p-[(R)-3-Aminobutyl]phenol

a) 2,50 g α,α'-[[[(R)-3-(p-Hydroxyphenyl)-1 -methylpropyl]imino]dimethylen]bis[(RS)-6-chlor -2-pyridinmethanol],

$$[\alpha]_D^{20^\circ} = +62^\circ \text{ (c = 0,7 in MeOH) und}$$

b) 3,5 g (RS)-6-Chlor-α-[[[(R)-3-(p-hydroxyphenyl) -1-methylpropyl]amino]methyl]-2-pyridinmethanol,

$$[\alpha]_D^{20°} = +4° \text{ (c = 0,6 in MeOH)}$$

2F) aus 2-Chlor-6-epoxyäthylpyridin und p-[(R)-2-Aminopropyl]benzoesäuremethylester

a) p-[(R)-2-[[(S)-2-(6-Chlor-2-pyridyl) -2-hydroxyäthyl]amino]propyl]benzoesäuremethylester, Smp. 140-142° (aus Methylenchlorid-Hexan),

$$[\alpha]_D^{20°} = +38° \text{ (c = 0,4 in MeOH)}$$

b) p-[(R)-2-[[(R)-2-(6-Chlor-2-pyridyl) -2-hydroxyäthyl]amino]propyl]benzoesäuremethylester, Smp. 67-68° (aus Aether),

$$[\alpha]_D^{20°} = -73° \text{ (c = 0,7 in MeOH) und}$$

c) p-[(R)-2-[Bis[(RS)-2-(6-chlor-2-pyridyl)-2 -hydroxyäthyl]amino]propyl]benzoesäuremethylester,

$$[\alpha]_D^{20°} = -33° \text{ (c = 0,3 in Methanol)}$$

2G) aus 2-Chlor-6-epoxyäthylpyridin und p-[(R)-2-Aminopropyl]-β-methylzimtsäuremethylester

a) p-[(R)-2-[[(R)-2-(6-Chlor-2-pyridyl) -2-hydroxyäthyl]amino]propyl] -β-methylzimtsäuremethylesterоxalat, Smp. 127-129°,

$$[\alpha]_D^{20°} = -39° \text{ (c = 0,9 in Methanol)}$$

b) p-[(R)-2-[[(S)-2-(6-Chlor-2-pyridyl) -2-hydroxyäthyl]amino]propyl] -β-methyl-zimtsäuremethylester, Smp. 101-102°,

$$[\alpha]_D^{20°} = -41° \text{ (c = 0,4 in MeOH) und}$$

c) p-[(R)-2-[Bis[(RS)-2-(6-chlor-2-pyridyl) -2-hydroxyäthyl]amino]propyl]-β-methylzimtsäuremethylester,

$$[\alpha]_D^{20°} = -26° \text{ (c = 0,3 in MeOH),}$$

2H) aus 1,0 g (R)-1-Methyl-3-(4-aminocarbonylphenyl)propylamin und 695 mg 2-(Epoxyäthyl)pyridin

a) 200 mg α,α'-[[[(R)-3-(p-Carbamoylphenyl)-1 -methylpropyl]imino]dimethylen]bis[(RS)-2-pyridinmethanol],

$$[\alpha]_D^{20°} = -48° \text{ (c = 1,0 in MeOH) und}$$

b) 829 mg p-[(R)-3-[[(RS)-β-Hydroxy-2-pyridyläthyl]amino]butyl]benzamid,

$$[\alpha]_D^{20^\circ} = +10^\circ \text{ (c = 1,0 in MeOH)},$$

2I) aus 1,0 g (R)-1-Methyl-3-(4-aminocarbonylphenyl)propylamin und 695 mg 4-(Epoxyäthyl)pyridin 480 mg p-[(R)-3-[[(RS)-β-Hydroxy-4-pyridyläthyl]amino]butyl]benzamid,

$$[\alpha]_D^{20^\circ} = +6^\circ \text{ (c = 1,0 in MeOH)}.$$

Beispiel 3

Eine Lösung von 500 mg (RS)-α-[[[(R)-p-Hydroxy-α -methylphenäthyl]amino]methyl]-2-pyridinmethanol (Beispiel 1b), 280 mg 2-Aethoxyäthylmethansulfonat und 185 mg KOH in 20 ml n-Propanol wurde 24 Stunden unter Argon zum Rückfluss erhitzt. Zur Aufarbeitung wurde auf Eiswasser gegossen und mit Aethylacetat extrahiert. Die organische Phase wurde mit Wasser gewaschen, mit $Na_2SO_4$ getrocknet und im Vakuum zur Trockene eingedampft. Der Rückstand wurde auf $SiO_2$ chromatographiert. Mit Chloroform/n-Propanol/wäss. ges. $NH_3$-Lösung (1000:20:2) wurden 340 mg (RS)-α-[[[(R)-p-(2--Aethoxyäthoxy)-α -methylphenäthyl]amino]methyl]-2-pyridinmethanol eluiert,

$$[\alpha]_D^{20^\circ} = -20^\circ \text{ (c = 0,3 in Methanol)}.$$

Beispiel 4

Analog Beispiel 3 erhielt man

4A) aus 600 mg α,α'-[[[(R)-p-Hydroxy-α -methylphenäthyl]imino]dimethylen]bis[(RS)-2-pyridinmethanol] (Beispiel 1a) und 282 mg 2-Aethoxyäthylmethansulfonat 325 mg α,α'-[[[(R)-p-(2-Aethoxyäthoxy)-α -methylphenäthyl]imino]dimethylen]bis[(RS) -2-pyridinmethanol],

$$[\alpha]_D^{20^\circ} = -23^\circ \text{ (c = 0,5 in Methanol)},$$

4B) aus 810 mg α,α'-[[[(R)-p-Hydroxy-α -methylphenäthyl]imino]dimethylen]bis[(RS)-6 -chlor-2-pyridinmethanol] (Beispiel 2Aa) und 336 mg 2-Aethoxyäthylmethansulfonat 450 mg α,α'-[[[(R)-p-(2-Aethoxyäthoxy)-α -methylphenäthyl]imino]dimethylen]bis[(RS)-6-chlor -2-pyridinmethanol],

$$[\alpha]_D^{20^\circ} = -23^\circ \text{ (c = 0,5 in MeOH)},$$

4C) aus 1,0 g (RS)-α-[[[(R)-p-Hydroxy-α -methylphenäthyl]amino]methyl]-6-chlor-2-pyridinmethanol (Beispiel 2Ab) und 610 mg 2-Aethoxyäthylmethansulfonat 900 mg (RS)-6-Chlor--α-[[[(R)-p-(2-äthoxyäthoxy)-α-methylphenäthyl]amino]methyl]-2-pyridinmethanol,

$$[\alpha]_D^{20^\circ} = -19^\circ \text{ (c = 1,0 in MeOH)},$$

4D) aus 1,23 g (RS)-α-[[[p-Hydroxyphenäthyl]amino]methyl]-2-pyridinmethanol (Beispiel 2Bb) und 0,95 g 2-Aethoxyäthylmethansulfonat

a) 0,64 g (RS)-α-[[[p-(2-Aethoxyäthoxy)phenäthyl]amino]methyl] -2-pyridinmethanol, IR-Banden bei 3296, 1611, 1591, 1571, 1511 $cm^{-1}$ und

b) 280 mg (RS)-α-[[(2-Aethoxyäthyl)[p-(2 -äthoxyäthoxy)phenäthyl]amino]methyl]-2-pyridinmethanol,

IR-Banden bei 3414, 1610, 1590, 1571, 1511, 1246, 1120, 1065, 823, 770 $cm^{-1}$.

4E) aus 780 mg α,-α'-[[[p-Hydroxyphenäthyl]imino]dimethylen]bis[(RS) -2-pyridinmethanol] (Beispiel 2Ba) und 368 mg 2-Aethoxyäthylmethansulfonat 540 mg α,α'-[[[p-(2-Aethoxyäthoxy)- phenäthyl]imino]-dimethylen]bis[(RS) -2-pyridinmethanol, IR-Banden bei 3364, 3233, 1610, 1591, 1571, 1511, 1245, 1123, 1067, 823, 771 $cm^{-1}$

4F) aus 830 mg (RS)-6-Chlor-α-[[[p-hydroxyphenäthyl]amino]methyl] -2-pyridinmethanol (Beispiel 2Cb) und 584 mg 2-Aethoxyäthylmethansulfonat

a) 500 mg (RS)-6-chlor-α-[[[p-(2-äthoxyäthoxy)phenäthyl]amino]methyl] -2-pyridinmethanol, IR-Banden bei 2927, 2870, 1610, 1583, 1561, 1511, 1438, 1247, 1121, 800 $cm^{-1}$ und

b) 252 mg (RS)-6-Chlor-α-[(2-äthoxyäthyl)[[p-(2 -äthoxyäthoxy)phenäthyl]amino]methyl]-2-pyridinmethanol, IR-Banden bei 3410, 2868, 1611, 1584, 1561, 1511, 1246, 1124, 1065, 824, 799 $cm^{-1}$

4G) aus 1,0 g α,α'-[[[p-Hydroxyphenäthyl]imino]dimethylen]bis[(RS) -6-chlor-2-pyridinmethanol] (Beispiel 2Ca) und 433 mg 2-Aethoxyäthylmethansulfonat 520 mg α,α'-[[[p-(2-Aethoxyäthoxy)phenäthyl]imino]-dimethylen]bis[(RS)-6-chlor-2-pyridinmethanol], IR-Banden bei 3385, 1610, 1584, 1561, 1511, 1245, 1157, 1133, 825, 799 $cm^{-1}$

4H) aus 1,80 g (RS)-α-[[[(R)-p-Hydroxy-α -methylphenäthyl]amino]methyl]-6-brom-2-pyridinmethanol (Beispiel 2Db) und 1,14 g 2-Aethoxyäthylmethansulfonat 1,1 g (RS)-6-Brom-α-[[[(R)-p-(2-äthoxyäthoxy)-α -methylphenäthyl]amino]methyl]-2-pyridinmethanol, Smp. 71° (aus Aceton-Hexan),

$$[\alpha]_D^{20°} = -5° \ (c = 0,5 \text{ in Methanol})$$

4I) aus 6,7 g α,α'-[[[(R)-p-Hydroxy-α -methylphenäthyl]imino]dimethylen]bis[(RS)-6-brom -2-pyridinmethanol] (Beispiel 2Da) und 2,35 g 2-Aethoxyäthylmethansulfonat

a) 2,0 g (R)-6-Brom-α-[[[(RS)-2-(6-brom-2-pyridyl)-2-hydroxyäthyl][(R)-p-(2-äthoxyäthoxy)-α -methylphenäthyl]amino]methyl]-2-pyridinmethanol,

$$[\alpha]_D^{20°} = -61° (c = 1,0 \text{ in MeOH}),$$

Diastereomeren-Verhältnis RSR:RRR = 2:1 und

b) 1,0 g α,α'-[[[(R)-p-(2-Aethoxyäthoxy-α -methylphenäthyl]imino]dimethylen]bis-[(S)-6-brom -2-pyridinmethanol],

$$[\alpha]_D^{20} = +58° \ (c = 1,0 \text{ in MeOH})$$

4J) aus 2,3 g α,α'-[[[(R)-p-Hydroxy-α -methylphenäthyl]imino]dimethylen]bis[(RS)-6 -chlor-2-pyridinmethanol] (Beispiel 2Aa) und 1,47 g 2-Phenäthoxyäthylmethansulfonat

a) 360 mg α,α'-[[[(R)-p-(2-Phenäthoxyäthoxy)-α-methylphenäthyl]imino]dimethylen]bis[(R)-6-chlor -2-pyridinmethanol],

$$[\alpha]_D^{20°} = -94° \ (c = 1,0 \text{ in MeOH})$$

b) 590 mg (R)-6-Chlor-α-[[[(S)-2-(6-chlor-2-pyridyl)-2-hydroxyäthyl][(R)-α-methyl-p -(2-phenäthoxyäthoxy)phenäthyl]amino]methyl] -2-pyridinmethanol,

$$[\alpha]_D^{20°} = -48° (c = 1,0 \text{ in MeOH}) \text{ und}$$

c) 520 mg α,α'-[[[(R)-p-[2-(Phenäthoxy)äthoxy]-α-methylphenäthyl]imino]dimethylen]bis[(S)-6-chlor -2-pyridinmethanol],

$$[\alpha]_D^{20°} = +31° \ (c = 1,0 \text{ in MeOH})$$

4K) aus 3,0 g α,α'-[[[(R)-p-Hydroxy-α -methylphenäthyl]imino]dimethylen]bis[(RS)-6-chlor -2-pyridinmethanol] (Beispiel 2Aa)

a) 869 mg des 1:2 Gemisches von α,α'-[[[(R)-p-(2-Aethoxyäthoxy)-α -methylphenäthyl]imino]-dimethylen]bis[(R)-6-chlor -2-pyridinmethanol] und (R)-α-[[[(S)-2-(6-Chlor-2-pyridyl)-2-hydroxyäthyl][-(R) -p-(2-äthoxyäthoxy)-α-methylphenäthyl]amino]methyl] -2-pyridinmethanol,

$$[\alpha]_D^{20°} = -62° \ (c = 0,3 \text{ in Methanol}) \text{ und}$$

b) 280 mg α,α'-[[[(R)-p-(2-Aethoxyäthoxy)-α -methylphenäthyl]imino]dimethylen]bis[(S)-6-chlor -2-pyridinmethanol,

$$[\alpha]_D^{20°} = +43° \ (c = 0,4 \text{ in Methanol})$$

4L) aus 1,60 g (RS)-6-Chlor-α-[[[(R)-3-(p-hydroxyphenyl)-1-methylpropyl]amino]methyl]-2-pyridinmethanol (Beispiel 2Eb) 0,970 g (RS)-6-Chlor-α-[[[(R)-3-(2-äthoxyäthoxy)phenyl] -1-methylpropyl]amino]methyl]-2-pyridinmethanol, Smp. 66°,

$$[\alpha]_D^{20°} = +6° \ (c = 0,8 \text{ in MeOH}).$$

Beispiel 5

Eine Lösung von 2,15 g α,α'-[[[(R)-p-Hydroxy-α-methylphenäthyl]imino]dimethylen]bis[(RS)-6-chlor-2-pyridinmethanol] (Beispiel 2Aa) in 95 ml Aceton wurde nach Zugabe von 314 mg pulverisierter KOH, 860 mg Bromessigsäuremethylester und einer Spur Kaliumjodid 5 Stunden bei Raumtemperatur unter Argon gerührt. Zur Aufarbeitung wurde auf Eiswasser gegossen und mit Aethylacetat extrahiert. Der organische Extrakt wurde mit Wasser neutral gewaschen, mit Natriumsulfat getrocknet und im Vakuum eingedampft. Der Rückstand wurde auf 300 g $SiO_2$ chromatographiert. Mit Chloroform/Hexan/n-Propanol/ges. $NH_3$-Lösung (1000:1000:5:0,5) wurden zunächst

a) 600 mg [p-[(R)-2-[Bis[(RS)-2-(6-chlor-2-pyridyl)-2-hydroxyäthyl]amino]propyl]phenoxy]-essigsäuremethylester,

$$[\alpha]_D^{20°} = -70° \ (c = 1,0 \text{ in Methanol}) \text{ eluiert.}$$

b) Die weiteren Fraktionen lieferten 310 mg [p-[(R)-2-[Bis[(S)-2-(6-chlor-2-pyridyl) -2-hydroxyäthyl]-amino]propyl]phenoxy]essigsäuremethylester,

$$[\alpha]_D^{20°} = +17° (c = 1,0 \text{ in Methanol}).$$

Beispiel 6

Analog Beispiel 5 erhielt man aus α,α'-[[[(R)-3-(p-Hydroxyphenyl)-1 -methylpropyl]imino]dimethylen]bis[-(RS)-6-chlor -2-pyridinmethanol] (Beispiel 2Ea)

a) [p-[(R)-3-[Bis[(RS)-2-(6-chlor-2-pyridyl) -2-hydroxyäthyl]amino]butyl]phenoxy]essigsäuremethylester,

$$[\alpha]_D^{20^\circ} = -24^\circ \ (c = 0,5 \text{ in MeOH}), \text{ und}$$

b) [p-[(R)-3-[Bis[(R)-2-(6-chlor-2-pyridyl) -2-hydroxyäthyl]amino]butyl]phenoxy]essigsäuremethylester,

$$[\alpha]_D^{20^\circ} = -112^\circ \ (c = 0,2 \text{ in MeOH}).$$

Beispiel 7

Eine Mischung von 800 mg (RS)-6-Chlor-α-[[[p-(2-äthoxyäthoxy)phenäthyl]amino]methyl] -2-pyridinmethanol (Beispiel 4Fa), 40 ml DMSO und 912 mg 2-[p-(2,3-Epoxypropoxy)phenyl]acetamid wurde 18 Stunden unter Rühren auf 100° erwärmt. Das Reaktionsgemisch wurde im Vakuum zur Trockene eingedampft und der Rückstand auf Silicagel chromatographiert. Mit Chloroform/n-Propanol/ges. wäss. $NH_3$ (1000:20:2) konnten 690 mg 2-[p-[(RS)-3-[[(RS)-2-(6-Chlor-2-pyridyl) -2-hydroxyäthyl][p-(2-äthoxyäthoxy)phenäthyl]-amino] -2-hydroxypropoxy]phenyl]acetamid eluiert werden, IR-Banden bei 3347, 3203, 1668, 1611, 1584, 1561, 1511, 1246, 1124, 822, 800 $cm^{-1}$.

Beispiel 8

Analog Beispiel 7 erhielt man

A) unter Verwendung von 4'-(2,3-Epoxypropoxy)acetanilid aus 1,0 g (RS)-6-Chlor-α-[[[(R)-p-(2-äthoxyäthoxy)-α-methylphenäthyl]amino]methyl]-2-pyridinmethanol (Beispiel 4C) 530 mg 4'-[(RS)-3-[[(RS)-2-(6-Chlor-2-pyridyl)-2-hydroxyäthyl][(R) -p-(2-äthoxyäthoxy)-α-methylphenäthyl]amino] -2-hydroxypropoxy]-acetanilid,

$$[\alpha]_D^{20^\circ} = -39^\circ \ (c = 0,4 \text{ in MeOH})$$

B) unter Verwendung von 1,2-Epoxy-3-[p-[2-(phenäthoxy)äthoxy]phenoxy]propan aus 1,0 g (RS)-6-Chlor-α-[[[(R)-p-(2-äthoxyäthoxy) -α-methylphenäthyl]amino]methyl]-2-pyridinmethanol (Beispiel 4C) 260 mg 6-Chlor-α-[[[(R)-p-(2-äthoxyäthoxy)-α -methylphenäthyl][(RS)-2-hydroxy-3-[p-[2 -(phenäthoxy)äthoxy]-phenoxy]propyl]amino]methyl]-2-pyridinmethanol,

$$[\alpha]_D^{20^\circ} = -41^\circ \ (c = 0,3 \text{ in MeOH})$$

C) aus 500 mg p-[(R)-2-[[(R)-2-(6-Chlor-2-pyridyl)-2-hydroxyäthyl]amino]propyl]benzoesäuremethylester (Beispiel 2Fb) und 442 mg (S)-3-Chlorstyroloxid 280 mg p-[(R)-2-[[(R)-2-(6-Chlor-2-pyridyl)-2-hydroxyäthyl][(S)-m -chlor-β-hydroxyphenäthyl]amino]propyl]benzoesäuremethylester,

$$[\alpha]_D^{20^\circ} = -59^\circ \ (c = 0,5 \text{ in MeOH}).$$

Beispiel 9

Zu einer auf 50° erwärmten Lösung von 1,89 g (RS)-6-Chlor-α-[[[(R)-p-(2-äthoxyäthoxy)-α -methylphenäthyl]amino]methyl]-2-pyridinmethanol (Beispiel 4C) in 100 ml Chloroform gab man innert 30 Minuten portionenweise 1,56 g 4-Amino-3,5-dichlor-phenacylbromid und erwärmte anschliessend noch 20 Stunden zum Rückfluss. Das Reaktionsgemisch wurde dann im Vakuum zur Trockene eingeengt. Der Rückstand wurde in 75 ml Methanol gelöst, mit 25 ml Wasser versetzt und die Lösung wurde auf 5° abgekühlt. Eine Lösung von 400 mg Natriumborhydrid in 5 ml Wasser wurde bei 0-5° zugetropft und das

Reaktionsgemisch wurde 90 Minuten gerührt. Zur Aufarbeitung wurde auf Eiswasser gegossen und mit Methylenchlorid dreimal extrahiert. Die organischen Extrakte wusch man mit Wasser, trocknete mit $Na_2SO_4$ und dampfte im Vakuum ein. Man erhielt 2,9 g Rohprodukt, das auf 200 g Silicagel chromatographiert wurde. Mit Hexan/Aceton 4:1 konnten 850 mg (RS)-α-[[[(RS)-4-Amino-3,5-dichlor-β-hydroxyphenäthyl][(R) -p-(2-äthoxyäthoxy)-α-methylphenäthyl]amino]methyl]-6-chlor -2-pyridinmethanol eluiert wurden,

$$[\alpha]_D^{20^\circ} = -39^\circ \text{ (c = 0,5 in MeOH).}$$

Beispiel 10

Ein Gemisch von 900 mg (RS)-6-Chlor-α-[[[p-(2-äthoxyäthoxy)phenäthyl]amino]methyl] -2-pyridinmethanol (Beispiel 4Fa), 25 ml Acetonitril, 0,41 ml Aethyljodid und 262 mg Natriumcarbonat wurde unter Rühren 7 Stunden auf 50° erwärmt. Nach Zugabe von 0,21 ml Aethyljodid wurde 44 Stunden auf 50° erwärmt. Zur Aufarbeitung wurde das Reaktionsgemisch filtriert und das Filtrat im Vakuum zur Trockene verdampft. Der Rückstand wurde auf 50 g Silicagel chromatographiert. Mit Chloroform/n-Propanol/ges. wäss. $NH_3$ - (1000:10:1) konnten 700 mg (RS)-6-Chlor-α-[[äthyl[p-(2-äthoxyäthoxy)phenäthyl]-amino]methyl]-2-pyridinmethanol eluiert werden, IR-Banden bei 3426, 1611, 1584, 1562, 1511, 1246, 1127, 822, 800 $cm^{-1}$.

Beispiel 11

Analog Beispiel 10 wurden unter Verwendung von Methyljodid anstelle von Aethyljodid aus 870 mg (RS)-6-Chlor-α-[[[(R)-p-(2-äthoxyäthoxy)-α -methylphenäthyl]amino]methyl]-2-pyridinmethanol (Beispiel 4C) 580 mg (RS)-6-Chlor-α-[[[(R)-p-(2-äthoxyäthoxy)-α -methylphenäthyl]methyl]amino]methyl]-2-pyridinmethanol erhalten,

$$[\alpha]_D^{20^\circ} = -8{,}5^\circ \text{ (c = 0,4 in MeOH).}$$

Beispiel 12

Eine Mischung von 1.39 g α,α'-[[[(R)-p-Hydroxy-α -methylphenäthyl]imino]dimethylen]bis[(RS)-6-chlor-2 -pyridinmethanol] (Beispiel 2Aa), 600 mg 6-Brom-1-hexanol, 370 mg Kalium-t-butylat und 15 ml DMSO wurde 90 Minuten bei Raumtemperatur unter Argon gerührt. Zur Aufarbeitung wurde im Vakuum eingedampft und der Rückstand auf $SiO_2$ chromatographiert. Es wurden isoliert:

a) 440mg α,α'-[[[(R)-p-(6-Hydroxyhexyloxy)-α -methylphenäthyl]imino]dimethylen]bis[(RS)-6-chlor -2-pyridinmethanol,

$$[\alpha]_D^{20^\circ} = -18^\circ \text{ (c = 0,3 in Methanol) und}$$

b) 370 mg (RS)-6-Chlor-α-[[[(RS)-2-(6-chlor -2-pyridyl)-2-(6-hydroxyhexyloxy)äthyl][(R) -p-(6-hydroxyhexyloxy)-α-methylphenäthyl]amino]methyl] -2-pyridinmethanol,

$$[\alpha]_D^{20^\circ} = -21^\circ \text{ (c = 0,5 in MeOH).}$$

Beispiel 13

484 mg 3-[(RS)-2-Oxiranyl]pyridin und 549 mg Tyramin wurden in 10 ml Acetonitril 20 Stunden unter Rückfluss gekocht. Das Reaktionsgemisch wurde im Vakuum zur Trockene eingedampft und der Rückstand wurde mit MeOH an Kieselgel chromatographiert. Nach Entfärbung mit Aktivkohle und Kristallisation aus

Acetonitril erhielt man 200 mg (RS)-α-[[(p-Hydroxyphenäthyl)amino]methyl] -3-pyridinmethanol, Smp. 112-114°.

Beispiel 14

Analog Beispiel 13 erhielt man

A) aus 1,3 g p-(2-Aethoxyäthoxy)-phenäthylamin (hergestellt durch Reaktion des N-Carbobenzoxytyramins in DMSO in Gegenwart von Kaliumhydroxyd mit Aethoxyäthylmethansulfonat und katalytische Hydrierung des erhaltenen Benzyl-[p-(2-äthoxyäthoxy)]phenäthylcarbamates in Methanol in Gegenwart von Pd/C) und 726 mg 3-[(RS)-2-Oxiranyl]pyridin

a) 670 mg (RS)-α-[[[p-(2-Aethoxyäthoxy)phenäthyl]amino]methyl] -3-pyridinmethanol, $\epsilon_{224}$ = 11900, $\epsilon_{261}$ = 3200 und

b) 150 mg α,α'-[[[p-(2-Aethoxyäthoxy)phenäthyl]imino]dimethylen]bis [(RS)-3-pyridinmethanol], NMR (in $CDCl_3$) 1,23 ppm (t) $CH_2$-$CH_3$; 2,6-3,1 ppm (m) $CH_2N$. $CH_2Ar$; 3,59 ppm (q) $CH_2$-$CH_3$; 3,77 und 4,10 ppm (t) O-$CH_2$-$CH_2$-O; 4,75 ppm (m) CH-OH; 6,9; 7,1; 7,26; 7,7; 8,5 ppm (m) arom. H,

B) aus 309 mg 3-[(RS)-2-Oxiranyl]pyridin und 570 mg (R)-p-(2-Aethoxyäthoxy)-α-methylphenäthylamin - hergestellt durch Reaktion des (R)-p-Hydroxy-α-methylphenäthylamins mit Chlorameisensäurebenzylester in Dioxan und Wasser in Gegenwart von Natriumbicarbonat, Umsetzung des erhaltenen (R)-N-Carbobenzoxy-p-hydroxy-α-methylphenäthylamins in DMSO mit Chloräthyläthyläther und Kaliumhydroxid, gefolgt durch katalytische Hydrierung in MeOH in Gegenwart von Pd/C des erhaltenen (R)-Benzyl-[p-(2-äthoxyäthoxy)-α -methylphenäthylcarbamats] - 248 mg (RS)-α-[[[(R)-p-(2-Aethoxyäthoxy)-α -methylphenäthyl]amino]methyl]-3-pyridinmethanol,

$$[\alpha]_D^{20°} = -23,2° \text{ (0,4\% in MeOH).}$$

Beispiel 15

Analog Beispiel 13 erhielt man

A) (RS)-4-Chlor-α-[[[p-(2-äthoxyäthoxy)phenäthyl]amino]methyl]-2-pyridinmethanol, $\epsilon_{268}$ = 3880 und

B) α,α'-[[[p-(2-Aethoxyäthoxy)phenäthyl]imino]dimethylen]bis[(RS)-4-chlor-2-pyridinmethanol], $\epsilon_{262}$ = 6630, $\epsilon_{269}$ = 6380.

Das als Ausgangsmaterial verwendete 4-Chlor-2-(2-oxiranyl)pyridin, $\epsilon_{201}$ = 15240, $\epsilon_{263}$ = 2850, $\epsilon_{268}$ = 460, stellte man her durch Umsetzung von 4-Chlor-2-pyridinaldehyd in einem Gemisch von Methylenchlorid und 50%iger Natronlauge mit Trimethylsulfoniummethylsulfat.

Beispiel 16

Analog Beispiel 13 erhielt man (RS)-2-Chlor-α-[[[p-(2-äthoxyäthoxy)phenäthyl]amino]methyl]-4-pyridinmethanol, Smp. 76-78° C; $\epsilon_{201}$ = 25940, $\epsilon_{224}$ = 13300, $\epsilon_{263}$ = 3740, $\epsilon_{269}$ = 3690.

Das als Ausgangsmaterial verwendete 2-Chlor-4-(2-oxiranyl)pyridin, $\epsilon_{201}$ = 15080, $\epsilon_{265}$ = 2790, stellte man her durch Reduktion von 2-chlorisonicotinsäuremethylester mit Diisobutylaluminiumhydrid in Toluol und Umsetzung des erhaltenen 2-Chlorisonicotinaldehyds, Smp. 46-48° C; $\epsilon_{264}$ = 2810, $\epsilon_{200}$ = 9950, in Methylenchlorid/50% NaOH mit Trimethylsulfoniummethylsulfat.

Beispiel 17

A) Eine Lösung von 958 mg p-[(R)-2-[[(R)-2-(6-Chlor-2-pyridyl)-2-hydroxyäthyl]amino]propyl]-β-methylzimtsäuremethylester-oxalat (Beispiel 2Ga), 60 ml 5% methanolischem KOH und 10 ml Wasser wurden unter Rühren 3 Stunden unter Argon auf 50° erwärmt. Zur Aufarbeitung wurde mit Wasser verdünnt, mit 2N Salzsäure auf pH 5 eingestellt und mehrmals mit Essigester extrahiert. Die vereinigten Extrakte wurden getrocknet und im Vakuum eingedampft. Man erhielt 550 mg amorphes p-[(R)-2-[[(R)-2-(6-Chlor-2-pyridyl)-2-hydroxyäthyl]amino]propyl]-β-methylzimtsäure-hydrochlorid, $[\alpha]_D^{20}$ = -45° (c = 0,5 in MeOH).

B) Analog Beispiel 17A erhielt man aus 697 mg p-[(R)-2-[[ (S)-2-(6-Chlor-2-pyridyl)-2-hydroxyäthyl]-amino]propyl]benzoesäuremethylester 420 mg amorphes p-[(R)-2-[[(S)-2-(6-chlor-2-pyridyl)-2-hydroxyäthyl]amino]propyl]benzoesäure-hydrochlorid, $[\alpha]_D^{20}$ = +33° (c = 0,5 in MeOH).

Beispiel 18

Es werden in üblicher Weise Tabletten folgender Zusammensetzung hergestellt:

| | |
|---|---|
| Wirkstoff der Formel I, z.B. (RS)-6-Chlor-α-[[[(R)-p-(2-äthoxyäthoxy)-α-methylphenäthyl]amino]methyl]-2-pyridinmethanol | 250 mg |
| Lactose | 200 mg |
| Maisstärke | 300 mg |
| Maisstärkepaste | 50 mg |
| Calciumstearat | 5 mg |
| Dicalciumphosphat | 45 mg |

Im Gegensatz zu den vorliegenden Verbindungen der Formel I, die eine oder zwei Pyridinäthanolgruppierungen enthalten, enthalten die in EP-A-23385, -70133, -99707, -101069 und -164700 beschriebenen Verbindungen eine oder zwei Phenäthanolgruppierungen.

**Patentansprüche**

**Patentansprüche für folgende Vertragsstaaten : BE, CH, LI, DE, FR, GB, IT, LU, NL, SE**

**1.** Pyridinäthanolaminderivate der Formel

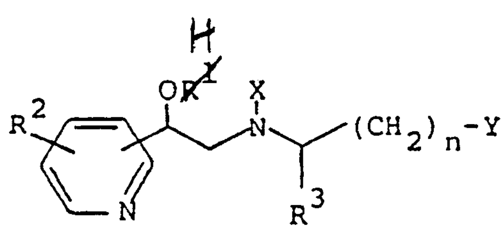

I

worin

| | |
|---|---|
| n | 1 oder 2 |
| X | H, nieder-Alkyl, nieder-Alkoxy-nieder-alkyl oder eine Gruppe $X^a$ der Formel |

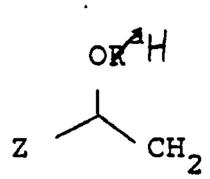

$X^a$

| | |
|---|---|
| Z | eine Gruppe der Formel |

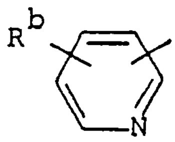

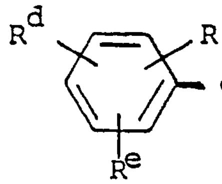

oder

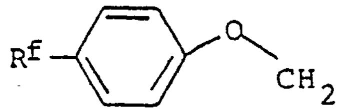

Z

($Z^1$) ($Z^2$) ($Z^3$)

Y eine Gruppe

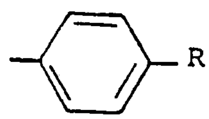

| | |
|---|---|
| R | eine Gruppe $COR^4$, $C(R^5)=CHCOR^4$ oder OR" |
| R" | H, $C_{1-4}$-Alkyl, $(CH_2)_{1-6}$-OH, $(CH_2)_{1-6}$-$O(CH_2)_{1-6}$-$R^6$ oder $(CH_2)_{1-6}$-$COR^4$ |
| $R^2$ und $R^b$ | H, Cl oder Br |
| $R^3$ und $R^5$ | H oder $CH_3$ |
| $R^4$ | Hydroxy, $C_{1-4}$-Alkoxy oder $NH_2$ |
| $R^6$ | H, $R^9$, OH oder $COR^4$ |
| $R^c$ und $R^e$ | H oder Cl, |
| $R^d$ | H oder $NH_2$ |
| $R^f$ | H, $CH_3CONH$, $NH_2COCH_2$ oder $R^9CH_2CH_2OCH_2CH_2O$ |
| $R^9$ und $R^9$ | Phenyl sind, |

sowie die physiologisch verträglichen Salze davon.

2. Verbindungen nach Anspruch 1, worin ein in der Gruppe Y vorliegender Rest $R^4$ $C_{1-4}$-Alkoxy oder $NH_2$ ist.

3. Verbindungen nach Anspruch 1 oder 2, worin n die Zahl 1, und $R^2$ Chlor in 6-Stellung eines 2-Pyridylrestes ist.

4. Verbindungen nach Anspruch 1, 2 oder 3, worin X Wasserstoff oder eine Gruppe $X^a$; Z 6-Chlor-2-pyridyl, und Y in p-Stellung durch 2-Aethoxyäthoxy, 2-Phenäthoxyäthoxy oder Methoxycarbonylmethoxy substituiertes Phenyl ist.

5. Verbindungen nach Anspruch 1, 2 oder 3, worin X eine Gruppe $X^a$; Z in p-Stellung durch Carbamoylmethyl, Acetamid oder 2-Phenäthoxyäthoxy substituiertes Phenoxymethyl, und Y p-(2-Aethoxyäthoxy)-phenyl ist.

6. Verbindungen nach Anspruch 1, 2, 3, 4 oder 5, worin $R^3$ Wasserstoff oder Methyl mit R-Konfiguration ist.

7. Verbindungen nach Anspruch 1 oder 2 aus der Gruppe der folgenden
   p-[(R)-2-[[(R)-2-(6-Chlor-2-pyridyl)-2-hydroxyäthyl]amino]propyl]benzoesäuremethylester,
   p-[(R)-2-[[(R)-2-(6-Chlor-2-pyridyl)-2-hydroxyäthyl][(S)-m-chlor-β-hydroxyphenäthyl]amino]-propylbenzoesäuremethylester,
   α,α'-[[[(R)-p-(2-Aethoxyäthoxy)-α-methylphenäthyl]imino]dimethylen]bis[(RS)-6-chlor-2-pyridinmethanol,
   (RS)-6-Chlor-α-[[[(R)-p-(2-äthoxyäthoxy)-α-methylphenäthyl]amino]methyl]-2-pyridinmethanol,
   α,α'-[[[p-(2-Aethoxyäthoxy)phenäthyl]imino]dimethylen]bis[(RS)-6-chlor-2-pyridinmethanol],
   (R)-6-Brom-α-[[[(RS)-2-(6-brom-2-pyridyl)-2-hydroxyäthyl][(R)-p-(2-äthoxyäthoxy)-α-methylphenäthyl]amino]methyl]-2-pyrimidinmethanol,
   (R)-6-Chlor-α-[[[(S)-2-(6-chlor-2-pyridyl)-2-hydroxyäthyl][(R)-α-methyl-p-(2-phenäthoxyäthoxy)-phenäthyl]amino]methyl]-2-pyridinmethanol,

2-[p-[(RS)-3-[[(RS)-2-(6-Chlor-2-pyridyl)-2-hydroxyäthyl][p-(2-äthoxyäthoxy)phenäthyl]amino]-2-hydroxypropoxy]phenyl]acetamid,

4'-[(RS)-3-[[(RS)-2-(6-Chlor-2-pyridyl)-2-hydroxyäthyl][(R)-p-(2-äthoxyäthoxy)-α-methylphenäthyl]-amino]-2-hydroxypropoxy]acetanilid und

6-Chlor-α-[[[(R)-p-(2-äthoxyäthoxy)-α-methylphenäthyl][(RS)-2-hydroxy-3-[p-[2-(phenäthoxy)äthoxy]-phenoxy]propyl]amino]methyl]-2-pyridinmethanol.

8. Verbindungen der Formel I nach einem der Ansprüche 1-7 und physiologisch verträgliche Salze davon als Mittel zur Behandlung der Fettsucht, des Diabetes mellitus und von Zuständen, die mit erhöhtem Proteinabbau verbunden sind, bzw. als Futterzusatz für Masttiere.

9. Pharmazeutische Präparate, gekennzeichnet durch einen Gehalt an einer Verbindung der Formel I nach einem der Ansprüche 1-7 oder an einem physiologisch verträglichen Salz davon.

10. Verfahren zur Herstellung von Verbindungen der Formel I nach einem der Ansprüche 1-7 und von physiologisch verträglichen Salzen davon, dadurch gekennzeichnet, dass man

a) ein Amin der Formel

$(X^1,X^2)NC(H,R^3)(CH_2)_nY$ II

worin eines von $X^1$ und $X^2$ Wasserstoff ist und das andere eine der Bedeutungen von X hat oder die Gruppe der Formel

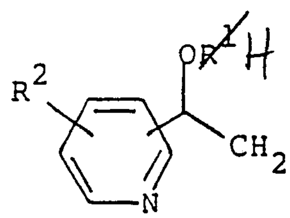

$X^3$

ist, mit einem die Gruppe $X^3$ oder eine der Gruppen X einführenden Mittel alkyliert und

b) gewünschtenfalls einen in einer Gruppe Y des Reaktionsproduktes enthaltenen reaktionsfähigen Substituenten funktionell abwandelt und gewünschtenfalls eine Verbindung der Formel I in ein Salz überführt.

11. Verwendung einer Verbindung der Formel I nach einem der Ansprüche 1-7 oder eines physiologisch verträglichen Salzes davon bei der Herstellung eines pharmazeutischen Präparates zur Behandlung der Fettsucht, des Diabetes mellitus und von Zuständen, die mit erhöhtem Proteinabbau verbunden sind, bzw. eines Futterzusatzes für Masttiere.

**Patentansprüche für folgende Vertragsstaaten : AT, ES, GR**

1. Verfahren zur Herstellung von Pyridinäthanolaminderivaten der Formel

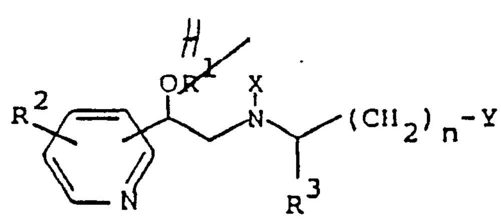

I

worin

n 1 oder 2

X H, nieder-Alkyl, nieder-Alkoxy-nieder-alkyl oder eine Gruppe $X^a$ der Formel

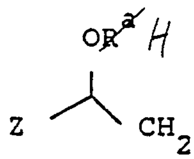

$X^a$

Z eine Gruppe der Formel

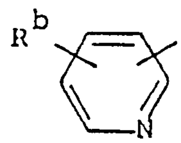

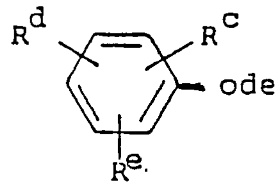

oder

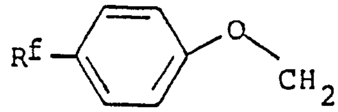

Z

($Z^1$) ($Z^2$) ($Z^3$)

Y eine Gruppe

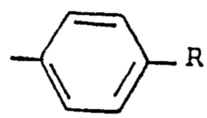

| | |
|---|---|
| R | eine Gruppe $COR^4$, $C(R^5)=CHCOR^4$ oder OR" |
| R" | H, $C_{1-4}$-Alkyl, $(CH_2)_{1-6}$-OH, $(CH_2)_{1-6}$-$O(CH_2)_{1-6}$-$R^6$ oder $(CH_2)_{1-6}$-$COR^4$ |
| $R^2$ und $R^b$ | H, Cl oder Br |
| $R^3$ und $R^5$ | H oder $CH_3$ |
| $R^4$ | Hydroxy, $C_{1-4}$-Alkoxy oder $NH_2$ |
| $R^6$ | H, $R^9$, OH oder $COR^4$ |
| $R^c$ und $R^e$ | H oder Cl, |
| $R^d$ | H oder $NH_2$ |
| $R^f$ | H, $CH_3CONH$, $NH_2COCH_2$ oder $R^9CH_2CH_2OCH_2CH_2O$ |
| $R^g$ und $R^9$ | Phenyl sind, |

sowie der physiologisch verträglichen Salzen davon, dadurch gekennzeichnet, dass man

a) ein Amin der Formel

$(X^1,X^2)NC(H,R^3)(CH_2)_nY$ II

worin eines von $X^1$ und $X^2$ Wasserstoff ist und das andere eine der Bedeutungen von X hat oder die Gruppe der Formel

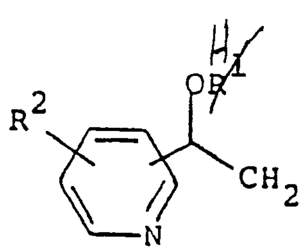

$X^3$

ist, mit einem die Gruppe $X^3$ oder eine der Gruppen X einführenden Mittel alkyliert und

b) gewünschtenfalls einen in einer Gruppe Y des Reaktionsproduktes enthaltenen reaktionsfähigen Substituenten funktionell abwandelt und gewünschtenfalls eine Verbindung der Formel I in ein Salz überführt.

2. Verfahren nach Anspruch 1, worin ein in der Gruppe Y vorliegender Rest $R^4$ $C_{1-4}$-Alkoxy oder $NH_2$ ist.

3. Verfahren nach Anspruch 1 oder 2, worin n die Zahl 1 und $R^2$ Chlor in 6-Stellung eines 2-Pyridylrestes ist.

4. Verfahren nach Anspruch 1, 2 oder 3, worin X Wasserstoff oder eine Gruppe $X^a$; Z 6-Chlor-2-pyridyl, und Y in p-Stellung durch 2-Aethoxyäthoxy, 2-Phenäthoxyäthoxy oder Methoxycarbonylmethoxy substituiertes Phenyl ist.

5. Verfahren nach Anspruch 1, 2 oder 3, worin X eine Gruppe $X^a$; Z in p-Stellung durch Carbamoylmethyl, Acetamid oder 2-Phenäthoxyäthoxy substituiertes Phenoxymethyl, und Y p-(2-Aethoxyäthoxy)phenyl ist.

6. Verfahren nach Anspruch 1, 2, 3, 4 oder 5, worin $R^3$ Wasserstoff oder Methyl mit R-Konfiguration ist.

7. Verfahren nach Anspruch 1 oder 2, dadurch gekennzeichnet, dass man eine Verbindung aus der Gruppe der folgenden herstellt:
p-[(R)-2-[[(R)-2-(6-Chlor-2-pyridyl)-2-hydroxyäthyl]amino]propyl]benzoesäuremethylester,
p-[(R)-2-[[(R)-2-(6-Chlor-2-pyridyl)-2-hydroxyäthyl][(S)-m-chlor-β-hydroxyphenäthyl]amino]propyl]-benzoesäuremethylester,
α,α'-[[[(R)-p-(2-Aethoxyäthoxy)-α-methylphenäthyl]imino]dimethylen]bis[(RS)-6-chlor-2-pyridinmethanol,
(RS)-6-Chlor-α-[[[(R)-p-(2-äthoxyäthoxy)-α-methylphenäthyl]amino]methyl]-2-pyridinmethanol,
α,α'-[[[p-(2-Aethoxyäthoxy)phenäthyl]imino]dimethylen]bis[(RS)-6-chlor-2-pyridinmethanol],
(R)-6-Brom-α-[[[(RS)-2-(6-brom-2-pyridyl)-2-hydroxyäthyl][(R)-p-(2-äthoxyäthoxy)-α-methylphenäthyl]amino]methyl]-2-pyrimidinmethanol,
(R)-6-Chlor-α-[[[(S)-2-(6-chlor-2-pyridyl)-2-hydroxyäthyl][(R)-α-methyl-p-(2-phenäthoxyäthoxy)-phenäthyl]amino]methyl]-2-pyridinmethanol,
2-[p-[(RS)-3-[[(RS)-2-(6-Chlor-2-pyridyl)-2-hydroxyäthyl][p-(2-äthoxyäthoxy)phenäthyl]amino]-2-hydroxypropoxy]phenyl]acetamid,
4'-[(RS)-3-[[(RS)-2-(6-Chlor-2-pyridyl)-2-hydroxyäthyl][(R)-p-(2-äthoxyäthoxy)-α-methylphenäthyl]-amino]-2-hydroxypropoxy]acetanilid und
6-Chlor-α-[[[(R)-p-(2-äthoxyäthoxy)-α-methylphenäthyl][(RS)-2-hydroxy-3-[p-[2-phenäthoxy)äthoxy]-phenoxy]propyl]amino]methyl]-2-pyridinmethanol.

8. Verfahren zur Herstellung von Arzneimitteln, insbesondere zur Behandlung der Fettsucht, des Diabetes mellitus und von Zuständen, die mit erhöhtem Proteinabbau verbunden sind, dadurch gekennzeichnet, dass man eine Verbindung der Formel I oder ein physiologisch verträgliches Salz davon in eine galenische Darreichungsform bringt.

9. Verwendung einer Verbindung der Formel I nach einem der Ansprüche 1-7 oder eines physiologisch verträglichen Salzes davon bei der Herstellung eines pharmazeutischen Präparates zur Behandlung der Fettsucht, des Diabetes mellitus und von Zuständen, die mit erhöhtem Proteinabbau verbunden sind, bzw. eines Futterzusatzes für Masttiere.

**Claims**
**Claims for the following Contracting States : BE, CH, LI, DE, FR, GB, IT, LU, NL, SE**

1. Pyridineethanolamine derivatives of the formula

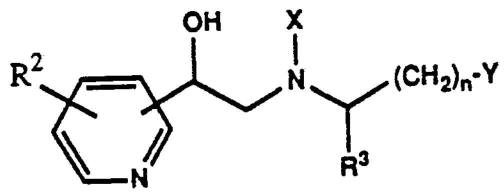

I

wherein

| | |
|---|---|
| n | is 1 or 2 |
| X | is H, lower-alkyl, lower-alkoxy-lower-alkyl or a group $X^a$ of the formula |

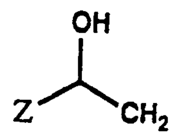

$X^a$

| | |
|---|---|
| Z | is a group of the formula |

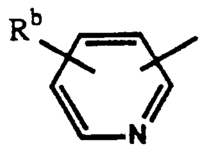

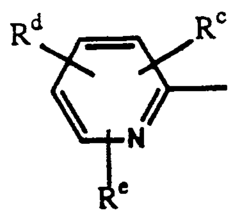

or

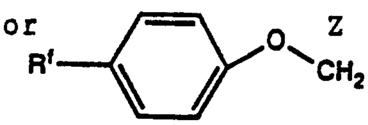

($Z^1$) ($Z^2$) ($Z^3$)

| | |
|---|---|
| Y | is a group |

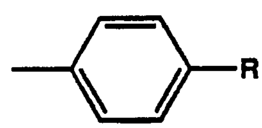

| | |
|---|---|
| R | is a group $COR^4$, $C(R^5)=CHCOR^4$ or OR", |
| R" | is H, $C_{1-4}$-alkyl, $(CH_2)_{1-6}$-OH, $(CH_2)_{1-6}$-$O(CH_2)_{1-6}$-$R^6$ or $(CH_2)_{1-6}$-$COR^4$ |
| $R^2$ and $R^b$ | are H, Cl or Br |
| $R^3$ and $R^5$ | are H or $CH_3$ |
| $R^4$ | is hydroxy, $C_{1-4}$-alkoxy or $NH_2$ |
| $R^6$ | is H, $R^9$, OH or $COR^4$ |
| $R^c$ and $R^e$ | are H or Cl |
| $R^d$ | is H or $NH_2$ |
| $R^f$ | is H, $CH_3CONH$, $NH_2COCH_2$ or $R^9CH_2CH_2OCH_2CH_2O$ |
| $R^9$ and $R^9$ | are phenyl, |

as well as the physiologically compatible salts thereof.

2. Compounds according to claim 1, wherein a residue $R^4$ present in group Y is $C_{1-4}$-alkoxy or $NH_2$.

3. Compounds according to claim 1 or 2, wherein n is the number 1 and $R^2$ is chlorine in the 6-position of a 2-pyridyl residue.

4. Compounds according to claim 1, 2, or 3, wherein X is hydrogen or a group $X^a$; Z is 6-chloro-2-pyridyl, and Y is phenyl substituted in the p-position by 2-ethoxyethoxy, 2-phenoxyethoxy or methoxycarbonylmethoxy.

5. Compounds according to claim 1, 2, or 3, wherein X is a group $X^a$; Z is phenoxymethyl substituted in the p-position by carbamoylmethyl, acetamide or 2-phenoxyethoxy, and Y is p-(2-ethoxyethoxy)phenyl.

6. Compounds according to claim 1, 2, 3, 4 or 5, wherein $R^3$ is hydrogen or methyl with the R-configuration.

7. Compounds according to claim 1 or 2 from the following group:
Methyl P-[(R)-2-[[(R)-2-(6-chloro-2-pyridyl)-2-hydroxyethyl]amino]propyl]benzoate,
methyl p-[(R)-2-[[(R)-2-(6-chloro-2-pyridyl)-2-hydroxyethyl][(S)-m-chloro-β-hydroxyphenethyl]-amino]propyl]benzoate,
α,α'-[[[(R)-p-(2-ethoxyethoxy)-α-methylphenethyl]imino]dimethylene]bis[(RS)-6-chloro-2-pyridinemethanol,
(RS)-6-chloro-α-[[[(R)-p-(2-ethoxyethoxy)-α-methylphenethyl]amino]methyl]-2-pyridinemethanol,
α,α'-[[[p-(2-ethoxyethoxy)phenethyl]imino]dimethylene]bis[(RS)-6-chloro-2-pyridinemethanol],
(R)-6-bromo-α-[[[(RS)-2-(6-bromo-2-pyridyl)-2-hydroxyethyl][(R)-p-(2-ethoxyethoxy)-α-methylphenethyl]amino]methyl]-2-pyrimidinemethanol,
(R)-6-chloro-α-[[[(S)-2-(6-chloro-2-pyridyl)-2-hydroxyethyl][(R)-α-methyl-p-(2-phenethoxyethoxy)-phenethyl]amino]methyl]-2-pyridinemethanol,
2-[p-[(RS)-3-[[(RS)-2-(6-chloro-2-pyridyl)-2-hydroxyethyl] [p-(2-ethoxyethoxy)phenethyl]amino]-2-hydroxypropoxy]phenyl]acetamide,
4'-[(RS)-3-[[(RS)-2-(6-chloro-2-pyridyl)-2-hydroxyethyl][(R)-p-(2-ethoxyethoxy)-α-methylphenethyl]-amino]-2-hydroxypropoxy]acetanilide and
6-chloro-α-[[[(R)-p-(2-ethoxyethoxy)-α-methylphenethyl][(RS)-2-hydroxy-3-[p-[2-(phenethoxy)-ethoxy]phenoxy]propyl]amino]methyl]-2-pyridinemethanol.

8. Compounds of formula I according to any one of claims 1-7 and physiologically compatible salts thereof as agents for the treatment of obesity, of diabetes mellitus and of conditions which are associated with an increased protein breakdown, or as feed additives for fattening animals.

9. Pharmaceutical preparations, characterized by a content of a compound of formula I according to any one of claims 1-7 or of a physiologically compatible salt thereof.

10. A process for the manufacture of compounds of formula I according to any one of claims 1-7 and of physiologically compatible salts thereof, characterized by
a) alkylating an amine of the formula

$(X^1,X^2)NC(H,R^3)(CH_2)_nY$ II

wherein one of $X^1$ and $X^2$ is hydrogen and the other has one of the significances of X or is the group of the formula

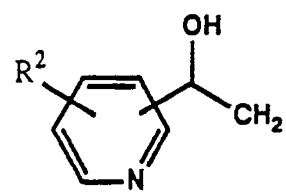

$X^3$

with an agent which introduces the group $X^3$ or one of the groups X and
b) if desired functionally modifying a reactive substituent present in a group Y of the reaction product and, if desired, converting a compound of formula I into a salt.

11. The use of a compound of formula I according to any one of claims 1-7 or of a physiologically compatible salt thereof in the manufacture of a pharmaceutical preparation for the treatment of obesity, of diabetes mellitus and of conditions which are associated with an increased protein breakdown, or of a feed additive for fattening animals.

**Claims for the following Contracting States : AT, ES, GR**

1. A process for the manufacture of pyridineethanolamine derivatives of the formula

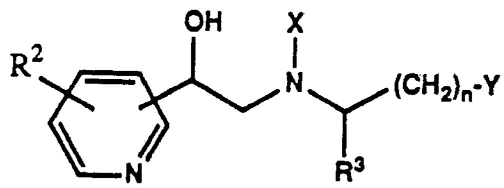

I

wherein

| | |
|---|---|
| n | is 1 or 2 |
| X | is H, lower-alkyl, lower-alkoxy-lower-alkyl or a group $X^a$ of the formula |

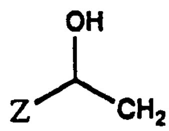

$X^a$

| | |
|---|---|
| Z | is a group of the formula |

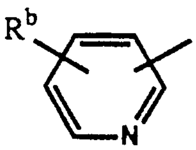

$(Z^1)$

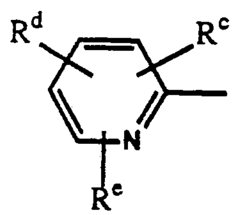

$(Z^2)$

or

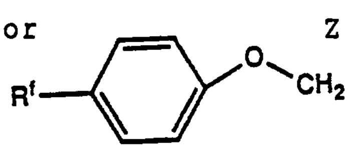

$(Z^3)$

| | |
|---|---|
| Y | is a group |

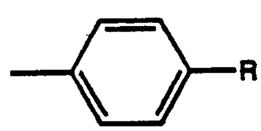

| | |
|---|---|
| R | is a group $COR^4$, $C(R^5)=CHCOR^4$ or OR", |
| R" | is H, $C_{1-4}$-alkyl, $(CH_2)_{1-6}$-OH, $(CH_2)_{1-6}$-$O(CH_2)_{1-6}$-$R^6$ or $(CH_2)_{1-6}$-$COR^4$ |
| $R^2$ and $R^b$ | are H, Cl or Br |
| $R^3$ and $R^5$ | are H or $CH_3$ |
| $R^4$ | is hydroxy, $C_{1-4}$-alkoxy or $NH_2$ |
| $R^6$ | is H, $R^9$, OH or $COR^4$ |
| $R^c$ and $R^e$ | are H or Cl |
| $R^d$ | is H or $NH_2$ |

$R^f$ is H, $CH_3CONH$, $NH_2COCH_2$ or $R^9CH_2CH_2OCH_2CH_2O$

$R^9$ and $R^9$ are phenyl,

as well as the physiologically compatible salts thereof, characterized by

a) alkylating an amine of the formula

$(X^1,X^2)NC(H,R^3)(CH_2)_nY$ II

wherein one of $X^1$ and $X^2$ is hydrogen and the other has one of the significances of X or is the group of the formula

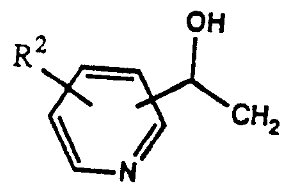

$X^3$

with an agent which introduces the group $X^3$ or one of the groups X and

b) if desired functionally modifying a reactive substituent present in a group Y of the reaction product and, if desired, converting a compound of formula I into a salt.

**2.** A process according to claim 1, wherein a residue $R^4$ present in group Y is $C_{1-4}$-alkoxy or $NH_2$.

**3.** A process according to claim 1 or 2, wherein n is the number 1 and $R^2$ is chlorine in the 6-position of a 2-pyridyl residue.

**4.** A process according to claim 1, 2, or 3, wherein X is hydrogen or a group $X^a$; Z is 6-chloro-2-pyridyl, and Y is phenyl substituted in the p-position by 2-ethoxyethoxy, 2-phenoxyethoxy or methoxycarbonylmethoxy.

**5.** A process according to claim 1, 2, or 3, wherein X is a group $X^a$; Z is phenoxymethyl substituted in the p-position by carbamoylmethyl, acetamide or 2-phenoxyethoxy, and Y is p-(2-ethoxyethoxy)phenyl.

**6.** A process according to claim 1, 2, 3, 4 or 5, wherein $R^3$ is hydrogen or methyl with the R-configuration.

**7.** A process according to claim 1 or 2, characterized in that a compound from the following group:

Methyl p-[(R)-2-[[(R)-2-(6-chloro-2-pyridyl)-2-hydroxyethyl]amino]propyl]benzoate,

methyl p-[(R)-2-[[(R)-2-(6-chloro-2-pyridyl)-2-hydroxyethyl][(S)-m-chloro-β-hydroxyphenethyl]-amino]propyl]benzoate,

α,α'-[[[(R)-p-(2-ethoxyethoxy)-α-methylphenethyl]imino]dimethylene]bis[(RS)-6-chloro-2-pyridinemethanol,

(RS)-6-chloro-α-[[[(R)-p-(2-ethoxyethoxy)-α-methyl-phenethyl]amino]methyl]-2-pyridinemethanol,

α,α'-[[[p-(2-ethoxyethoxy)phenethyl]imino]dimethylene]bis[(RS)-6-chloro-2-pyridinemethanol],

(R)-6-bromo-α-[[[(RS)-2-(6-bromo-2-pyridyl)-2--hydroxyethyl][(R)-p-(2-ethoxyethoxy)-α-methylphenethyl]amino]methyl]-2-pyrimidinemethanol,

(R)-6-chloro-α-[[[(S)-2-(6-chloro-2-pyridyl)-2-hydroxyethyl][(R)-α-methyl-p-(2-phenethoxyethoxy)-phenethyl]amino]methyl]-2-pyridinemethanol,

2-[p-[(RS)-3-[[(RS)-2-(6-chloro-2-pyridyl)-2-hydroxyethyl][p-(2-ethoxyethoxy)phenethyl]amino]-2-hydroxypropoxy]phenyl]acetamide,

4'-[(RS)-3-[[(RS)-2-(6-chloro-2-pyridyl)-2-hydroxyethyl][(R)-p-(2-ethoxyethoxy)-α-methylphenethyl]-amino]-2-hydroxypropoxy]acetanilide and

6-chloro-α-[[[(R)-p-(2-ethoxyethoxy)-α-methylphenethyl][(RS)-2-hydroxy-3-[p-[2-(phenethoxy)-ethoxy]phenoxy]propyl]amino]methyl]-2-pyridinemethanol

is manufactured.

**8.** A process for the manufacture of medicaments, especially for the treatment of obesity, of diabetes mellitus and of conditions which are associated with an increased protein breakdown, characterized by

bringing a compound of formula I or a physiologically compatible salt thereof into a galenical administration form.

9. The use of a compound of formula I according to any one of claims 1-7 or of a physiologically compatible salt thereof in the manufacture of a pharmaceutical preparation for the treatment of obesity, of diabetes mellitus and of conditions which are associated with an increased protein breakdown, or of a feed additive for fattening animals.

**Revendications**

**Revendications pour les Etats contractants suivants : BE, CH, LI, DE, FR, GB, IT, LU, NL, SE**

1. Dérivés de pyridineéthanolamine de formule

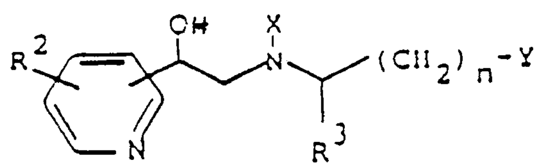

où

n vaut 1 ou 2

X représente H, alcoyle inférieur- alcoxy inférieur- ou un groupe $X^a$ de formule

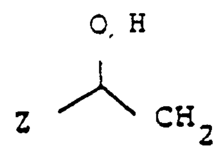

$X^a$

Z représente un groupe de formule

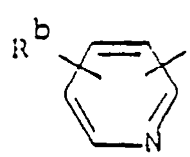

(z1)

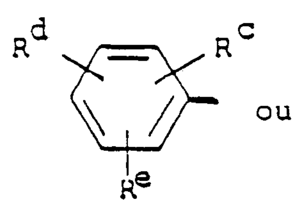

(z2)

ou

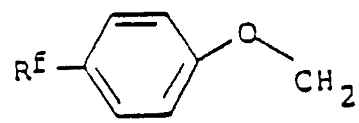

(z3)

Z

Y représente un groupe

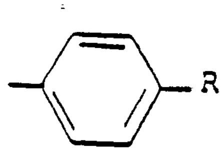

R représente un groupe $COR^4$, $C(R^5)=CHCOR^4$ ou OR"

R" représente H, alcoyle en $C_{1-4}$, $(CH_2)_{1-6}$-OH, $(CH_2)_{1-6}$-O$(CH_2)_{1-6}$-$R^6$ ou $(CH_2)_{1-6}$-$COR^4$,

$R^2$ et $R^b$ représentent H, Cl ou Br
$R^3$ et $R^5$ représentent H ou $CH_3$,
$R^4$ représente un hydroxy, alcoxy en $C_{1-4}$ ou $NH_2$
$R^6$ représente H, $R^g$, OH ou $COR^4$
$R^c$ et $R^e$ représentent H ou Cl,
$R^d$ représente H ou $NH_2$,
$R^f$ représente H, $CH_3CONH$, $NH_2COCH_2$ ou $R^9CH_2CH_2OCH_2CH_2O$,
$R^g$ et $R^9$ représentent un phényle,

ainsi que de leurs sels physiologiquement acceptables

2. Composés selon la revendication 1, où un radical $R^4$ présent dans le groupe Y est un alcoxy en $C_{1-4}$ ou $NH_2$.

3. Composés selon les revendications 1 ou 2, où n représente le nombre 1 et $R^2$ représente un chlore en position 6 d'un radical 2-pyridyle.

4. Composés selon les revendications 1, 2 ou 3, où X représente un hydrogène ou un groupe $X^a$ ; Z représente un 6-chloro-2-pyridyle, et Y est un phényle substitué en position p par un 2-éthoxyéthoxy, 2-phénéthoxyéthoxy ou méthoxycarbonyleméthoxy.

5. Composés selon les revendications 1, 2 ou 3, où X est un un groupe $X^a$ ; Z est un phénoxyméthyle substitué en position p par un carbamoyleméthyle, acétamide ou 2-phénéthoxyéthoxy et Y est un p-(2-éthoxyéthoxy)phényle.

6. Composés selon les revendications 1, 2, 3, 4 ou 5 où $R^3$ représente un hydrogène ou un méthyle avec la configuration R.

7. Composés selon la revendication 1 ou 2, du groupe des composés suivants
Ester méthylique de l'acide p-[(R)2-[[(R)-2-(6-chloro-2-pyridyl)-2-hydroxyéthyl]amino]propyl]-benzoïque,
Ester méthylique de l'acide p-[(R)-2-[[R)-2-(6-chloro-2-pyridyl)-2-hydroxyéthyl][(S)-m-chloro-$\beta$-hydroxyphénéthyl]amino]propyl]benzoïque,
$\alpha,\alpha'$-[[[(R)-p-(2-éthoxyéthoxy)-$\alpha$-méthylphénéthyl]imino]diméthylèn]bis[(RS)6-chloro-2-pyridineméthanol,
(RS)-6-chloro-$\alpha$-[[[(R)-p-(2-éthoxyéthoxy)-$\alpha$-méthyl-phénéthyl]-amino]méthyl]-2-pyridineméthanol,
$\alpha,\alpha'$-[[[p-(2-éthoxyéthoxy)phénéthyl]imino]diméthylèn]bis[(RS)-6-chloro-2-pyridineméthanol],
(R)-6-bromo-$\alpha$-[[[(RS)-2-(6-bromo-2-pyridyl)-2-hydroxy-éthyl][(R)-p-2-(éthoxyéthoxy)-$\alpha$-méthylphénéthyl]amino]méthyl]-2-pyridineméthanol,
(R)-6-chloro-$\alpha$-[[[(S)-2-(6-chloro-2-pyridyl)-2-hydroxy-éthyl][(R)-$\alpha$-méthyl-p- (2-phénéthoxyéthoxy)-phénéthyl]-amino]-méthyl]-2-pyridineméthanol,
2-[p-[(RS)-3-[[(RS)-2-(6-chloro-2-pyridyl)-2-hydroxy-éthyl][p-(2-éthoxyéthoxy)phénéthyl]amino]-2-hydroxypropoxy]-phényl]acétamide,
4'-[(RS)-3-[[(RS)-2-(6-chloro-2-pyridyl)-2-hydroxyéthyl]-[(R)-p-(2-éthoxyéthoxy)-$\alpha$-méthylphénéthyl]-amino]-2-hydroxy-propoxy]acétanilide et
6-chloro-$\alpha$-[[[(R)-p-(2-éthoxyéthoxy)-$\alpha$-méthylphénéthyl][(RS)-2-hydroxy-3-[p-[2-(phénéthoxy)-éthoxy]phénoxy]-propyl]amino]méthyl]-2-pyridineméthanol.

8. Composés de formule I selon l'une des revendications 1-7 et leurs sels physiologiquement acceptables comme agents pour le traitement de l'obésité, du diabète sucré et des états qui sont caractérisés par une dégradation accrue des protéines ou comme additifs de fourrage pour les animaux à l'engrais.

9. Préparations pharmaceutiques, caractérisées en ce qu'elles contiennent un composé de formule I selon l'une des revendications 1-7 ou un de leurs sels physiologiquement acceptables.

10. Procédé de préparation de composés de formule I selon l'une des revendications 1-7 et de leurs sels physiologiquement acceptables, caractérisé en ce que
a) on alcoyle une amine de formule

$(X^1,X^2)NC(H,R^3)(CH_2)_nY$ II

où l'un des radicaux $X^1$ et $X^2$ est un hydrogène et l'autre a l'une des significations de X ou est le groupe de formule

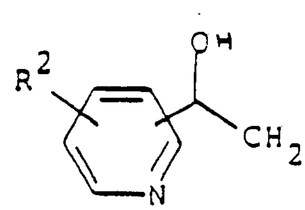

$X^3$

avec un agent introduisant le groupe $X^3$ ou l'un des groupes X, et

b) si on le désire, on transforme de façon fonctionnelle un substituant réactif contenu dans un groupe Y du produit de la réaction, et si on le désire on transforme un composé de formule I en un sel.

**11.** Application d'un composé de formule I selon l'une des revendications 1-7 ou d'un de ses sels physiologiquement acceptables à la préparation d'une préparation pharmaceutique pour le traitement de l'obésité, et des états qui sont caractérisés par une dégradation accrue des protéines, ou d'un additif de fourrage pour animaux à l'engrais.

**Revendications pour les Etats contractants suivants : AT, ES, GR**

**1.** Procédé de préparation de dérivés de pyridinéthanolamine de formule

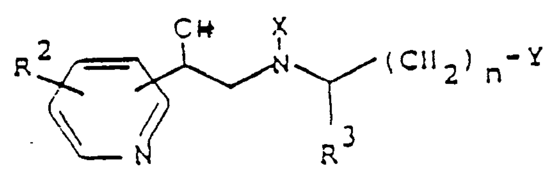

I

où

n vaut 1 ou 2

X représente H, alcoyle inférieur- alcoxy inférieur- ou un groupe $X^a$ de formule

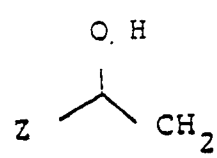

$X^a$

Z représente un groupe de formule

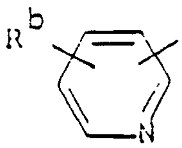

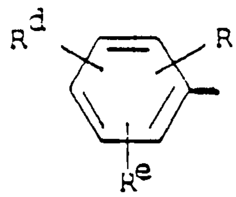

ou

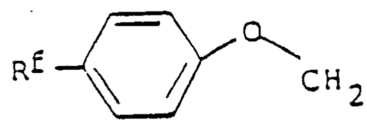

Z

($Z^1$) ($Z^2$) ($Z^3$)

Y représente un groupe

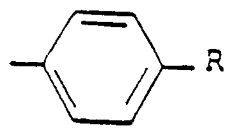

R représente un groupe $COR^4$, $C(R^5)=CHCOR^4$ ou OR"
R" représente H, alcoyle en $C_{1-4}$, $(CH_2)_{1-6}$-OH, $(CH_2)_{1-6}$-$O(CH_2)_{1-6}$-$R^6$ ou $(CH_2)_{1-6}$-$COR^4$,
$R^2$ et $R^b$ représentent H, Cl ou Br
$R^3$ et $R^5$ représentent H ou $CH_3$,
$R^4$ représente un hydroxy, alcoxy en $C_{1-4}$ ou $NH_2$
$R^6$ représente H, $R^9$, OH ou $COR^4$
$R^c$ et $R^e$ représentent H ou Cl,
$R^d$ représente H ou $NH_2$,
$R^f$ représente H, $CH_3CONH$, $NH_2COCH_2$ ou $R^9CH_2CH_2OCH_2CH_2O$,
$R^9$ et $R^9$ représentent un phényle,

ainsi que de leurs sels physiologiquement acceptables, caractérisés en ce que

a) on alcoyle une amine de formule

$(X^1,X^2)NC(H,R^3)(CH_2)_nY$ II

ou l'un des radicaux $X^1$ et $X^2$ représente un hydrogène et l'autre a l'une des significations de X ou est le groupe de formule

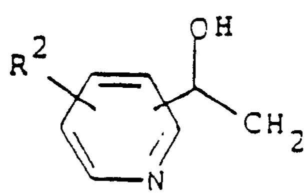

$X^3$

avec un groupe $X^3$ ou un agent introduisant un des groupes X, et

b) si on le désire, on transforme fonctionnellement un substituant réactif contenu dans un groupe Y du produit de la réaction, et si on le désire on transforme un produit de formule I en un sel.

2. Procédé selon la revendication 1, dans lequel un radical $R^4$ présent dans le groupe Y est un alcoxy en $C_{1-4}$ ou $NH_2$.

3. Procédé selon les revendications 1 ou 2, où n représente le nombre 1 et $R^2$ représente un chlore en position 6 d'un radical 2-pyridyle.

4. Procédé selon les revendications 1, 2 ou 3, où X représente un oxygène ou un groupe $X^a$; Z représente un 6-chloro-2-pyridyle, et Y représente un phényle substitué en position p par un 2-éthoxyéthoxy, 2-phééthoxyéthoxy ou méthoxycarbonyleméthoxy.

5. Procédé selon les revendications 1, 2 ou 3, où X représente un groupe $X^a$; Z représente un phénoxyméthyle substitué en position p par un carbamoylméthyle, acétamide ou 2 phéthoxyéthoxy, et Y est un p-(2-éthoxyéthoxy)phényle.

6. Procédé selon les revendications 1, 2, 3, 4 ou 5 où $R^3$ est un hydrogène ou un méthyle avec une configuration R.

7. Procédé selon les revendications 1 ou 2, caractérisé en ce qu'on prépare un composé du groupe des composés suivants :

Ester méthylique de l'acide p-[(R)2-[[(R)-2-(6-chloro-2-pyridyl)-2-hydroxyéthyl]-amino]propyl]-benzoïque,

Ester méthylique de l'acide p-[(R)-2-[[R)-2-(6-chloro-2-pyridyl)-2-hydroxyéthyl][(S)-m-chloro-β-hydroxyphénéthyl]amino]propyl]benzoïque,

α,α'-[[[(R)-p-(2-éthoxyéthoxy)-α-méthylphénéthyl]imino]diméthylèn]bis[(RS)6-chloro-2-pyridineméthanol,

(RS)-6-chloro-α-[[[ (R)-p-(2-éthoxyéthoxy)-α-méthyl-phénéthyl]-amino]méthyl]-2-pyridineméthanol,

α,α'-[[[p-(2-éthoxyéthoxy)phénéthyl]imino]diméthylène]bis[(RS)-6-chloro-2-pyridineméthanol],

(R)-6-bromo-α-[[[(RS)-2-(6-bromo-2-pyridyl)-2-hydroxy-éthyl][(R)-p-2-(éthoxyéthoxy)-α-méthylphénéthyl]amino]méthyl]-2-pyridineméthanol,

(R)-6-chloro-α-[[[(S)-2-(6-chloro-2-pyridyl)-2-hydroxy-éthyl][(R)-α-méthyl-p-(2-phénéthoxyéthoxy)-phénéthyl]-amino]-méthyl]-2-pyridineméthanol,

2-(p-[(RS)-3-[[(RS)-2-(6-chloro-2-pyridyl)-2-hydroxy-éthyl][p-(2-éthoxyéthoxy)phénéthyl]amino]-2-hydroxypropoxy]-phényl]acétamide,

4'-((RS)-3-[[(RS)-2-(6-chloro-2-pyridyl)-2-hydroxyéthyl]-[(R)-p-(2-éthoxyéthoxy)-α-méthylphénéthyl]-amino]-2-hydroxy-propoxy]acétanilide et

6-chloro-α-[[[(R)-p-(2-éthoxyéthoxy-α-méthylphénéthyl][(RS)-2-hydroxy-3-[p-[2-(phénéthoxy)éthoxy]-phénoxy]-propyl]amino]méthyl]-2-pyridineméthanol.

8. Procédé de préparation de médicament, en particulier pour le traitement de l'obésité, du diabète sucré et des états caractérisés par une dégradation protéique accrue, caractérisé en ce qu'on met sous une forme d'administration galénique un composé de formule I ou un de ses sels physiologiquement acceptables.

9. Application d'un composé de formule I selon l'une des revendications 1-7 ou l'un de ses sels physiologiquement acceptables à la préparation d'une préparation pharmaceutique pour le traitement de l'obésité, du diabète sucré et des étapes qui sont liées à un accroissement de la dégradation protéique, ou d'un additif alimentaire pour les animaux à l'engrais.